# EUROPEAN PATENT APPLICATION

(11) **EP 2 644 097 A1**
(43) Date of publication of application: **02.10.2013**
(21) Application number: 10859963.0
(22) Date of filing: 09.12.2010
(51) Int. Cl.: A61B 8/00, G01N 29/24

(54) **PROBE HAVING SEPARABLE SCANHEAD**

(30) Priority: 23.11.2010 KR 20100116565
(71) Applicant: Alpinion Medical Systems Co., Ltd., Guro-gu, Seoul, 152-848 (KR)
(72) Inventor: KO, Seokbin, Seongnam-si Gyeonggi-do 463-919 (KR); NOH, Wonho, Seoul 137-932 (KR); LEE, Sang Woong, Yongin-si Gyeonggi-do 448-532 (KR); LEE, Jae Won, Incheon 405-230 (KR)
(74) Representative: Groth & Co. KB
(86) International application number: PCT/KR2010/008801
(87) International publication number: WO 2012/070711

(57) **Abstract**

The invention relates to a probe that is comprised in an ultrasonic diagnostic system, wherein the probe comprises: a scanhead which includes a housing that functions as a handle, a transducer that is installed on one side of the housing, a tuning board that is accommodated within the housing and has one side connected with a PCB of the transducer, and a board connection-connector that is connected with the other side of the tuning board; and a cable assembly which has one end on which a scanhead connector detachably coupled with the board-connection connector is positioned, and has the other end on which a system connector is placed. Since the scanhead can be separated from the cable assembly such that the scanhead can be replaced, overall convenience of a user is improved greatly, and consequently, the ultrasonic diagnostic system can be miniaturized and carried in a portable form.

## Description

### [Technical Field]

The present invention relates to a probe, and more particularly, to a probe which is provided in an ultrasonic diagnostic system and in which a scanhead can be separated.

### [Background Art]

In general, ultrasonic diagnostic systems are systems that irradiate an ultrasonic signal onto a target part inside an object to be inspected from a body surface of the object to be inspected, extract information from the reflected ultrasonic signal and obtain an image regarding a fault of a soft tissue or a blood stream in an noninvasive manner.

These ultrasonic diagnostic systems have advantages, such as smaller sizes and lower prices, real-time display, and high safety in which they have no irradiation exposure of X-ray, in comparison with other imaging diagnostic devices such as X-ray inspection devices, computerized tomography (CT) scanners, magnetic resonance image (MRI) scanners, or nuclear medicine inspection devices. Thus, ultrasonic diagnostic systems have been widely used to diagnose the heart, internal organs of the abdomen, an urinary system, and genital organs.

In particular, ultrasonic diagnosis systems include a probe that transmits an ultrasonic signal to an object to be inspected and receives the ultrasonic signal reflected from the object to be inspected so as to obtain an ultrasonic image of the object to be inspected.

The probe includes a scanhead including a handle and a transducer, a cable assembly that is connected to one side of the scanhead, and a system connector that is connected to an opposite side to the scanhead of the cable assembly and connects to an ultrasonic diagnostic system.

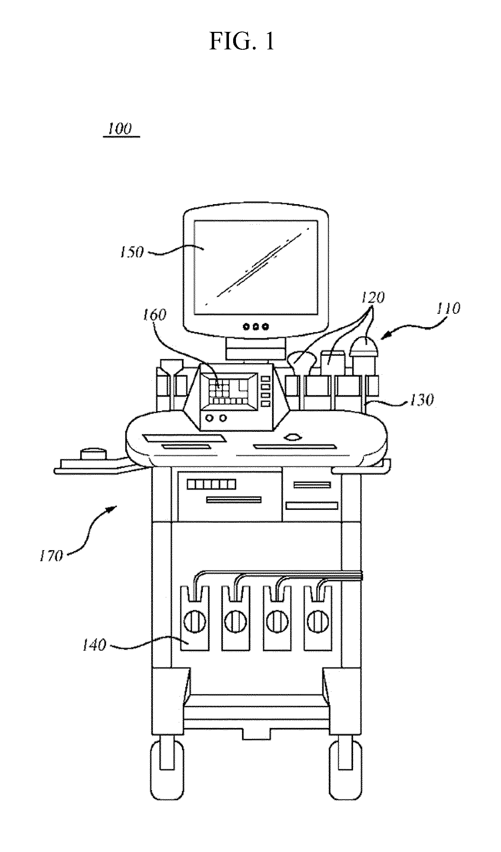

FIG. 1 illustrates an example of an ultrasonic diagnostic system according to the related art. Referring to FIG. 1, an ultrasonic diagnostic system 100 according to the related art includes various types of probes 110 provided according to their usages, a display unit 150 that displays a diagnosis result as an image, an input unit 160 including various manipulation buttons, switches, and a keyboard, and a main body unit 170 in which a central processing unit (CPU)(not shown) performing various arithmetic operations is embedded and which retains and supports components. Here, the main body unit 170 may also represent a portion other than the probe 110, the display unit 150, and the input unit 160 of the ultrasonic diagnostic system 100.

Each probe 110 according to the related art is formed integrally with a scanhead 120, a cable assembly 130 connected to the scanhead 120, and a system connector 140 that connects the cable assembly 130 to the main body unit 170.

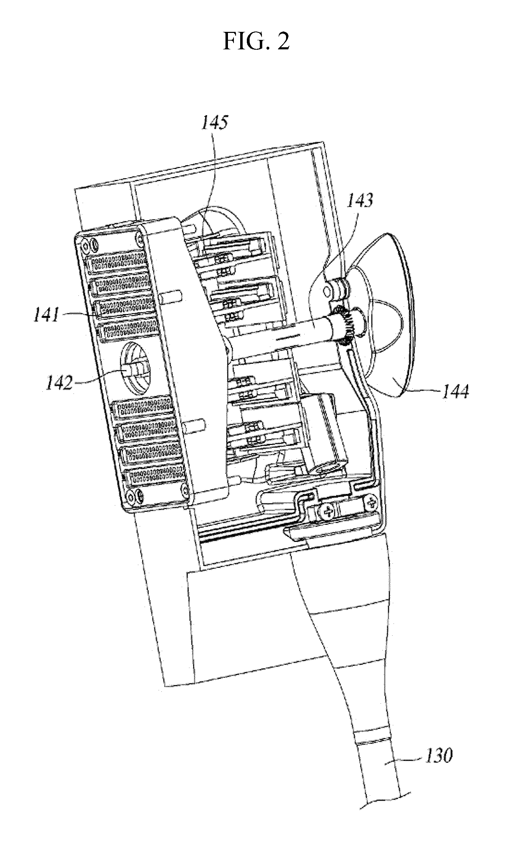

The system connector 140 includes a plurality of terminal parts 141 that are arranged at one side of the system connector 140 so that the system connector 140 can be connected to a socket (not shown) mounted in the main body unit 170, a fastening shaft 143 that passes through the system connector 140 and has a fastening pin 142 protruding from an outer circumferential surface of the fastening shaft 143 so as to be mechanically fastened to the socket of the main body unit 170, and a handle 144 that is provided at the other side of the system connector 140 so as to rotate the fastening shaft 143, as specifically illustrated in FIG. 2. Also, the system connector 140 further includes a tuning board 145 that is accommodated within the system connector 140 and has one side connected to the plurality of terminal parts 141 and the other side connected to a plurality of wires (not shown) of the cable assembly 130.

When the probe 110 is connected to the main body unit 170, the terminal parts 114 of the system connector 140 are inserted into the socket of the main body unit 170, the fastening shaft 143 is inserted into an insertion hole (not shown) of the socket, the handle 144 is rotated, the fastening pin 142 of the fastening shaft 143 is inserted into a fastening groove (not shown) of the main body unit 170 such that the system connector 140 can be fastened to the main body unit 170.

Since one probe 110 can generate only one type of ultrasonic wave having a designated frequency band, the ultrasonic diagnostic system 100 should include a plurality of probes 110 having frequency bands suitable for user's desired performance and diagnostic regions. Three or four probes 110 are generally mounted on the main body unit 170 according to performance and diagnostic regions, and each probe 110 has an unique identification (ID). The mounted probes 110 are retained in and supported by the above-described fastening unit in a state in which they are connected to the main body unit 170, and a user checks an ID of each probe 110 and then manipulates the input unit 160 so as to select one probe 110 to be used.

The cable assembly 130 is used in signal transmission/reception between the probe 110 and the main body unit 170. Input/output terminals in the socket of the main body unit 170 and the system connector 140 of the cable assembly 130 have different impedances. In order to improve sensitivity and a band width of the probe 110 by reducing signal reflection caused by an impedance difference, in the related art, the tuning board 145 is installed in the system connector 140 so as to match the impedances.

The user moves the scanhead 120 of the probe 110 having the above-described configuration along a body surface of an object to be inspected or rotates the scanhead 120 of the probe 110 in contact with the body surface so as to obtain a desired ultrasonic image.

Since the ultrasonic diagnostic system 100 is used in various medical fields, such as internal medicine, pediatrics, obstetrics and gynaecology and urology, the ultrasonic diagnostic system 100 according to the related art may additionally include various types of probes except the types of FIG. 1.

However, since, in the ultrasonic diagnostic system according to the related art, each probe is formed integrally with a scanhead, a cable assembly and a system connector, when a probe is replaced with a new one, there is inconvenience that the entire body of the probe should be replaced with a new one and connected.

Furthermore, since, in the ultrasonic diagnostic system according to the related art, the probe is integrally connected to the scanhead, the cable assembly and the system connector, a high-priced cable assembly or the like should be overlappingly purchased, the price of a product increases, and even when reusable elements for maintenance remain, all of the scanhead, the cable assembly, and the system connector should be discarded.

In addition, since the ultrasonic diagnostic system according to the related art should include a plurality of probes, a space in which the plurality of probes are to be installed, is required, and thus the size and weight of the entire system increase.

Meanwhile, in the ultrasonic diagnostic system according to the related art, the system connector has a complicated structure, and a large number of components, such as a tuning board, an ID recognition circuit, and the like, are embedded in the system connector, Thus, the volume of the system connector unnecessarily increases, which results in limitations in configuring the ultrasonic diagnostic system to be miniaturized and to be carried in a portable form.

### [Disclosure]

### [Technical Problem]

The present invention is directed to providing a separable probe in which various types of scanheads are connected to one cable assembly so that the entire cost of the probe can be reduced and the size and weight of an ultrasonic diagnostic system can be greatly reduced.

The present invention is also directed to providing a probe in which a system connector connected between a main body unit of an ultrasonic diagnostic system and a cable assembly is miniaturized so that the ultrasonic diagnostic system can be miniaturized and carried in a portable form.

### [Technical Solution]

One aspect of the present invention provides a probe including: a scanhead which includes a housing that functions as a handle, a transducer that is installed at one side of the housing, a tuning board that is accommodated within the housing and has one side connected to a printed circuit board (PCB) of the transducer, and a board-connection connector that is connected to the other side of the tuning board; and a cable assembly which has one end on which a scanhead connector detachably coupled to the board-connection connector is provided, and has the other end on which a system connector is provided.

### [Effect of the Invention]

As described above, in a probe according to the present invention, if necessary, a scanhead can be separated from a cable assembly such that the scanhead can be replaced with a new one and user's working convenience can be greatly improved. Furthermore, an optimized design suitable for each type of probe can be realized.

In addition, in a probe according to the present invention, since one cable assembly has only to be mounted on a main body unit of an ultrasonic diagnostic system, the entire structure of the main body unit is simplified, and a space in which the probe is to be installed, the size and weight of the entire system can be greatly reduced.

In addition, in a probe according to the present invention, a scanhead is replaceably provided so that an initial cost for purchasing a cable assembly is not overlappingly required, a manufacturing cost of a product can be reduced, and even when maintenance is required, reusable elements can be continuously used without discarding all of the scanhead, the cable assembly and a system connector, thus easily performing maintenance and reducing a cost for maintenance.

In a probe according to the present invention, the ultrasonic diagnostic system can be consequently miniaturized and carried in a portable form.

### [Description of Drawings]

FIG. 1 is a front view illustrating an example of an ultrasonic diagnostic system according to the related art;
FIG. 2 is a cross-sectional perspective view specifically illustrating a system connector included in the ultrasonic diagnostic system according to the related art;
FIG. 3 is an exploded perspective view of a probe according to an embodiment of the present invention;
FIG. 4 is an exploded perspective view of a probe according to another embodiment of the present invention;
FIGS. 5A and 5B are cross-sectional views specifically illustrating the usage state of a detachment unit illustrated in FIG. 4;
FIG. 6 is an exploded perspective view of a probe according to another embodiment of the present invention;
FIG. 7A is a cross-sectional view specifically illustrating a detachment unit illustrated in FIG. 6, and FIG. 7B is a cross-sectional view of a groove part of a sleeve;

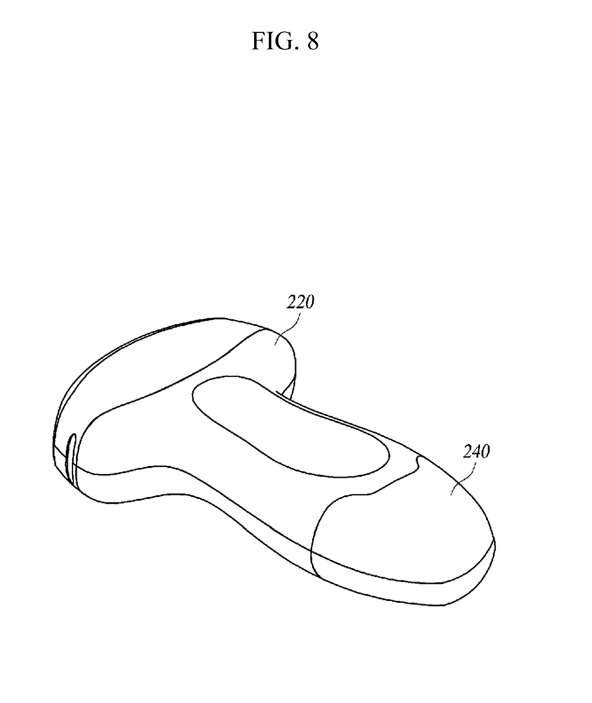

FIG. 8 is a perspective view illustrating a state in which a scanhead of a probe according to the present invention is separated from a cable assembly and is coupled to an additional cover;
FIG. 9 is a perspective view illustrating part of various types of probes configured according to the present invention; and
FIG. 10 illustrates an example of a state in which the probe according to the present invention is connected to an ultrasonic diagnostic system.

### [Modes of the Invention]

The present invention will now be described more fully with reference to the accompanying drawings, in which exemplary embodiments of the invention are shown. First, when adding reference numerals to elements of the drawings, it should be noted that like reference numerals are used for like elements if possible although like elements are shown in different drawings. In addition, in the description of the present invention, if it is determined that a detailed description of commonly-used configurations or functions related to the invention may unnecessarily obscure the subject matter of the invention, the detailed description will be omitted.

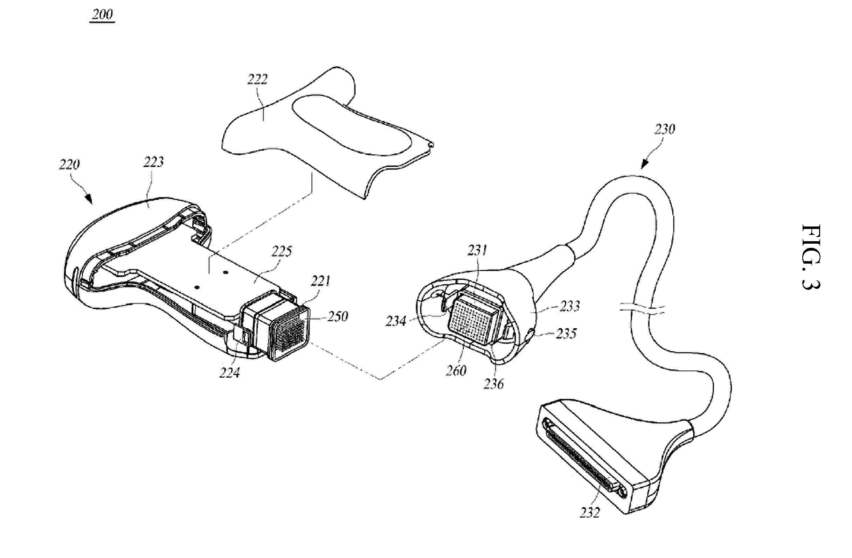

FIG. 3 is an exploded perspective view of a separable probe according to an embodiment of the present invention. Referring to FIG. 3, a probe 200 according to an embodiment of the present invention includes a scanhead 220 and a cable assembly 230. The scanhead 220 includes a board-connection connector 221. The cable assembly 230 includes a scanhead connector 231 provided on one end of the cable assembly 230 and a system connector 232 provided on the other end of the cable assembly 230.

The scanhead 220 of the probe 200 according to the current embodiment of the present invention is formed as an independent element that can be separated from the cable assembly 230 and can be electrically connected to the cable assembly 230 via the board-connection connector 221 and the scanhead connector 231.

The scanhead 220 includes a housing 222 that functions as a handle, a transducer 223 that is installed at one side of the housing 222, a tuning board 225 that is accommodated within the housing 222 and has one side connected to a printed circuit board (PCB)(not shown) of the transducer 223, and the board-connection connector 221 that is connected to the other side of the tuning board 225.

The housing 222 constitutes the exterior of the scanhead 220 and is ergonomically designed in such a way that a user can grip the housing 222 conveniently when the scanhead 220, i.e., the probe 200 is used. Thus, the shape of the scanhead 220 can be optimized suitable for each type of probe. A hollow space part in which an element such as the tuning board 225 that will be described below in detail can be accommodated, is formed in the housing 222.

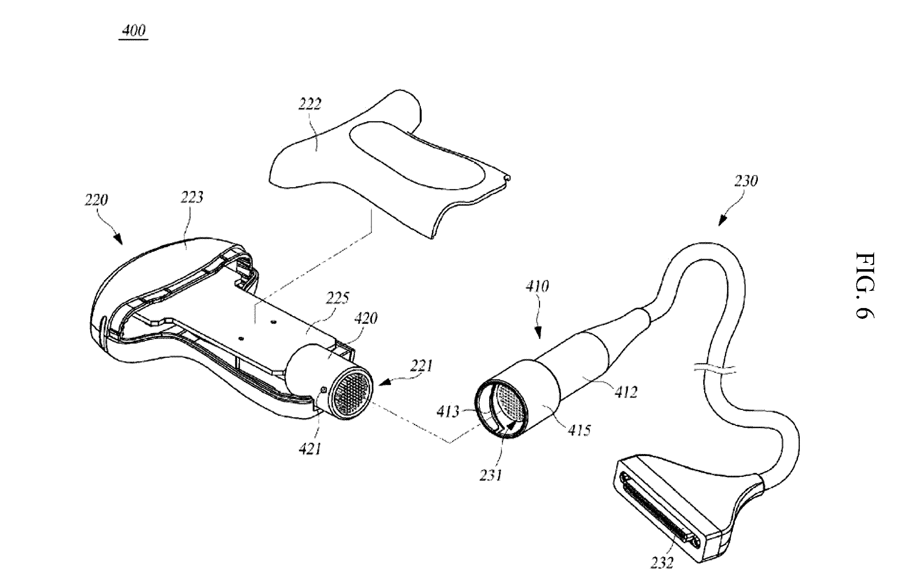

When the cable assembly 230 is separated from the scanhead 220, as illustrated in FIG. 6, the housing 222 may allow an additional storage cover 240 to be coupled to an open part of the housing 222. Thus, when the separated scanhead 220 is kept, foreign substances or moisture can be prevented from permeating into the housing 222, and elements inside the scanhead 220 can be protected.

Although the transducer 223 is not specifically illustrated for simplified illustration, the transducer 223 includes a piezoelectric layer in which a piezoelectric material vibrates so as to transform an electrical signal and an acoustic signal with respect to each other, a matching layer that reduces a difference in acoustic impedance between the piezoelectric layer and an object to be inspected so that an ultrasonic signal generated in the piezoelectric layer can be transmitted to the object to be inspected to the maximum, a lens layer that focuses the ultrasonic signal proceeding toward a front of the piezoelectric layer on a particular point, and a sound-absorbing layer that prevents image distortion by preventing an ultrasonic wave from proceeding toward a rear of the piezoelectric layer. A PCB is connected to the piezoelectric layer. Wiring electrodes are formed on the PCB so as to be connected to electrodes of the piezoelectric layer and thus the PCB serves to transmit a signal of the piezoelectric layer.

The transducer 223 of the probe 200 according to the current embodiment of the present invention irradiates an ultrasonic wave onto a target part and transforms the reflected ultrasonic wave into an electrical signal. The configuration and function of a transducer are substantially widely known to one of ordinary skill in the art and thus detailed descriptions thereof will be omitted.

The tuning board 225 accommodated within the housing 222 is a PCB in which an integrated circuit (IC) is highly integrated and mounted with high density. A connection electrode to be electrically connected to the PCB of the transducer 223 is included in one side of the tuning board 225, and the board-connection connector 221 is connected to and fixed to the other side of the tuning board 225. Connection between the tuning board 225 and the PCB of the transducer 223 may be performed by a first connector (not shown) connected to the PCB and a second connector (not shown) connected to one end of the tuning board 225. Each connector described above may be configured in such a way that, if one side connector is a plug connector, an opposite side connector is a socket connector so that corresponding connectors can be easily coupled to each other.

The tuning board 225 is configured to optimize characteristics of a signal by correcting the frequency of the signal transmitted from the transducer 223. To this end, the tuning board 225 may embed a signal processing circuit (not shown) including an inductor. That is, the tuning board 225 impedance-matches an electrically-received signal transmitted from the transducer 223 and outputs the matched received signal, matches an electrically-transmitted signal transmitted from a main body unit (see 170 of FIG. 1) via the cable assembly 230 and outputs the matched transmitted signal.

A tuning board according to the related art is embedded in the system connector (see 140 of FIG. 1) that connects the cable assembly 230 to the main body unit (see 170 of FIG. 1) of the ultrasonic diagnostic system and thus limits miniaturizing the size of the ultrasonic diagnostic system. However, in the probe 200 according to the current embodiment of the present invention, the tuning board 225 is installed in the scanhead 220 and thus the above-described limitation is overcome. Such an advantage is very useful in configuring the ultrasonic diagnostic system to be miniaturized and to be carried in a portable form.

Furthermore, an ID recognition circuit is provided integrally with the tuning board 225. Thus, when the scanhead 220 is connected to the cable assembly 230, the tuning board 225 outputs an ID signal that allows a probe to be recognized, i.e., an ID signal of the scanhead 220 to the main body unit (see 170 of FIG. 1) of the ultrasonic diagnostic system.

The board-connection connector 221 is connected to the tuning board 225 of the scanhead 220 and is exposed to an outer side of the housing 222. Here, the board-connection connector 221 and the tuning board 225 may be electrically connected to each other using a press-fit pin structure, a ball grid array package structure, a dip soldering coupling structure, or the like. The board-connection connector 221 connects the scanhead 220 and the cable assembly 230 and is detachably coupled to the scanhead connector 231 of the cable assembly 230.

Here, either one of the board-connection connector 221 and the scanhead connector 231 that can be coupled to each other includes a plurality of pin members 250 formed as conductors, and the other one thereof has an insertion hole 260 in which the plurality of pin members 250 are inserted and conductors through which the board-connection connector 221 and the scanhead connector 231 can be mechanically and electrically connected to each other, are embedded. In other words, if one side connector is a plug connector, an opposite side connector may be implemented as a socket connector. These connectors 221 and 231 are so-called high-density connectors and may be 50 pins or more.

A connection cover 233 extends from the cable assembly 230 so as to surround the periphery of the scanhead connector 231. An open part of the connection cover 233 needs to have a shape corresponding to the open part of the housing 222. Thus, corresponding stepped parts may be formed at front ends of the open parts in consideration of mutual fit and sealing. The connection cover 233 may be formed of a waterproof material, for example, silicon and may be manufactured using a method, such as double injection or insert injection. Furthermore, an outer side of the connection cover 233 may be waterproof-coated. Moreover, a rubber ring 236 or a gasket for preventing water permeation may be disposed in a portion in which the board-connection connector 221 and the scanhead connector 231 are coupled to each other. The stepped parts, the waterproof material, waterproof coating, and the rubber ring allow the probe 200 according to the current embodiment of the present invention to have waterproof performance, thus satisfying a permission criterion of a medical device.

As described above, the cable assembly 230 has one end on which the scanhead connector 231 is provided, and the other end on which the system connector 232 is provided. A connector or a socket (not shown) having a structure corresponding to the system connector 232 of the cable assembly 230 is mounted on the main body unit of the ultrasonic diagnostic system. These connectors 231 and 232 are so-called high-density connectors and may be 50 pins or more.

If the system connector 232 and the socket are completely coupled to each other, the probe 200 according to the current embodiment of the present invention is mechanically and electrically connected to the ultrasonic diagnostic system. Also, by connecting the board-connection connector 221 and the scanhead connector 231 and connecting the system connector 232 and the socket as described above, the tuning board 225 provided in the scanhead 220 is electrically connected to the main body unit of the ultrasonic diagnostic system via the cable assembly 230.

The structure and shape of each connector are not limited to FIG. 3, and various types of connectors may be used. However, connectors that are easily capable of being coupled to each other, satisfy reliability of coupling and are lightweight may be selected. Also, the connectors may include units for preventing misinsertion. For example, corresponding connectors may have approximately trapezoidal cross-sections, or at least one edge of each connector may be formed in a corresponding shape, or a protrusion or groove in a corresponding shape may be formed in a corresponding position of each connector.

The probe 200 according to the current embodiment of the present invention includes a detachment unit that enables the coupled scanhead 220 and cable assembly 230 or the board-connection connector 221 and the scanhead connector 231 not to be easily separated from each other by an arbitrary shock and to be coupled to or separated from each other only by user's manipulation.

Through the detachment unit, for example, in the current embodiment of the present invention, a pair of latches 234 are provided at the connection cover 233 that surrounds the scanhead connector 231, and simultaneously, a pair of hanging grooves 224 or hanging holes which are formed in the housing 222 in the vicinity of the board-connection connector 221 and in which the pair of latches 234 may be hung, are provided to correspond to the pair of latches 234.

The latches 234 are formed of an elastic material and may have an elastic restoring force perpendicular to a direction in which the board-connection connector 221 and the scanhead connector 231 are coupled to each other. Additionally, the latches 234 may include pressurization parts 235 that allow the latches 234 to elastically move by user's pressurization, and springs may be provided to elastically support the pressurization parts 235 and may be not necessary. Also, the latches 234 and the pressurization parts 235 may be formed integrally with each other or to be separated from each other. The pressurization parts 235 serve as separation switches that are used to separate the coupled scanhead 220 and cable assembly 230 or the board-connection connector 221 and the scanhead connector 231 from each other.

In the current embodiment of the present invention, the latches 234 are formed at the connection cover 233, and the hanging grooves 224 are formed in the housing 222. In contrast, the latches 234 may be formed at the housing 222, and the hanging groove 224 may be formed in the connection cover 233. Also, the shapes and sizes of the latches 234 and the hanging groove 224 may be modified in various ways.

The latches 234 are hung in and fixed to the hanging grooves 224 such that the board-connection connector 221 and the scanhead connector 231 are coupled to each other and the scanhead 220 and the cable assembly 230 are coupled to each other. Thus, the latches 234 and the hanging grooves 224 are hung in each other, and the board-connection connector 221 and the scanhead connector 231 are coupled to each other so that the scanhead 220 and the cable assembly 230 can be mechanically and electrically connected to each other with reliability.

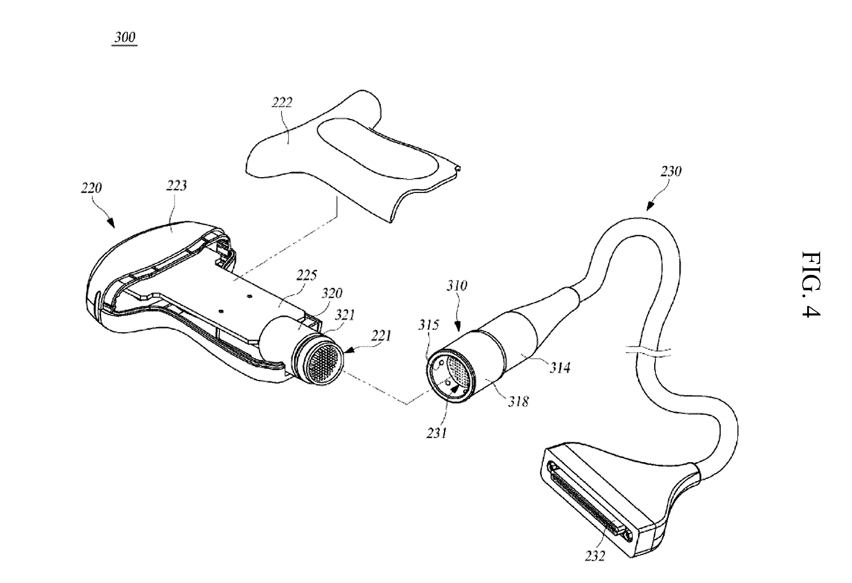

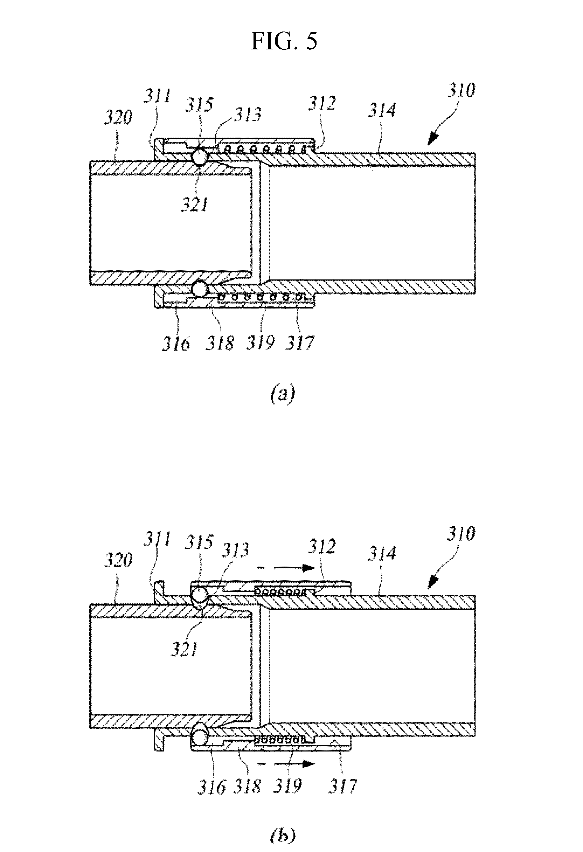

FIG. 4 is an exploded perspective view of a probe according to another embodiment of the present invention, and FIGS. 5A and 5B are cross-sectional views specifically illustrating the usage state of a detachment unit illustrated in FIG. 4.

Only a connection structure of a probe 300 according to another embodiment of the present invention is different from the probe 200 of FIG. 1, and the other portions of the probe 300 are the same as those of the probe 200 of FIG. 1. In the current embodiment of the present invention, a ball coupling unit 310 is provided to surround a scanhead connector 231 and to be connected to and fixed to one end of a cable assembly 230, and simultaneously, a snap coupling unit 320 which is installed in a housing 222 in the vicinity of a board-connection connector 221 and to which the ball coupling unit 310 can be fastened, is provided to correspond to the ball coupling unit 310.

The ball coupling unit 310 includes a tubular coupling body 314 which has one end connected to and fixed to the cable assembly 230 and has the other end on which an edge part 311 is formed and in which support protrusions 312 are formed on an outer circumferential surface of the coupling body 314 to be spaced apart from the edge part 311 and a plurality of through holes 313 are formed in a space between the edge part 311 and the support protrusions 312, ball members 315 that are mounted in the plurality of through holes 313 of the coupling body 314, a sleeve 318 including a first stepped part 316 and a second stepped part 317 while surrounding ends of the coupling body 314, and an elastic member 319 that is interposed between the second stepped part 317 of the sleeve 318 and an outer circumferential surface of the coupling body 314 and is elastically supported by the support protrusions 312.

The edge part 311 limits movement of the sleeve 318 when the sleeve 318 moves in a lengthwise direction of the coupling body 314. The support protrusions 312 are formed in a ring shape on the outer circumferential surface of the coupling body 314, support one side of the elastic member 319 and simultaneously limit movement of the sleeve 318. The edge part 311 or the support protrusions 312 may be omitted for easy assembling of the sleeve 318 and may be replaced with a C-shaped ring.

Diameters of the through holes 313 of the coupling body 314 toward an inner circumferential surface of the coupling body 314 are formed to be small, and diameters thereof toward the outer circumferential surface of the coupling body 314 are formed to be relatively large. Thus, the ball members 315 do not pass through the through holes 313 but are mounted in the through holes 313 while parts of the ball members 315 protrude toward the inner circumferential surface of the coupling body 314.

The first stepped part 316 of the sleeve 318 provides a clearance in which the ball members 315 are movable from the through holes 313 by a predetermined distance when coupling of the ball coupling unit 310 and the snap coupling unit 320 is released. Meanwhile, the second stepped part 317 serves as an accommodation groove in which the elastic member 319 is accommodated, and serves as a support part in which an end jaw part of the second stepped part 317 supports the other side of the elastic member 319. An end of the sleeve 318 toward the first stepped part 316 is required to sufficiently cover the through holes 313 of the coupling body 314 even when the user pulls the sleeve 318 manually toward an opposite side to the scanhead 220 so that the ball members 315 move to the maximum, thus preventing the ball members 315 from completely escaping from the through holes 313.

The snap coupling unit 320 is a tubular member in which the snap coupling unit 320 is inserted into an inner side of the coupling body 314 of the ball coupling unit 310 and a ring-shaped coupling groove 321 is formed adjacent to an end of the snap coupling unit 320 in a circumferential direction. A board-connection connector 221 is accommodated within the snap coupling unit 320.

Thus, if the snap coupling unit 320 is inserted into the coupling body 314 of the ball coupling unit 310, parts of the ball members 315 that protrude toward the inner circumferential surface of the coupling body 314 through the through holes 313 are inserted into the coupling groove 321 of the snap coupling unit 320 and thus the ball coupling unit 310 and the snap coupling unit 320 are coupled to and fixed to each other.

When coupling of the ball coupling unit 310 and the snap coupling unit 320 is released, if the user pulls the sleeve 318 toward the opposite side of the scanhead 220 manually, due to the first stepped part 316 of the sleeve 318, a clearance in which the ball members 315 are movable within the through holes 313 toward the outer circumferential surface of the coupling body 314, is generated so that the ball members 315 move in a radial direction and escape from the coupling groove 321 of the snap coupling unit 320. Thus, the snap coupling unit 320 can be separated from the ball coupling unit 310.

The ball members 315 are fastened to and fixed to the coupling groove 321 such that the board-connection connector 221 and the scanhead connector 231 are connected to each other and the scanhead 220 and the cable assembly 230 are coupled to each other. Thus, the ball members 315 are fastened to the coupling groove 321, and the board-connection connector 221 and the scanhead connector 231 are coupled to each other so that mechanical connection and electrical connection between the scanhead 220 and the cable assembly 230 can be conveniently and reliably performed.

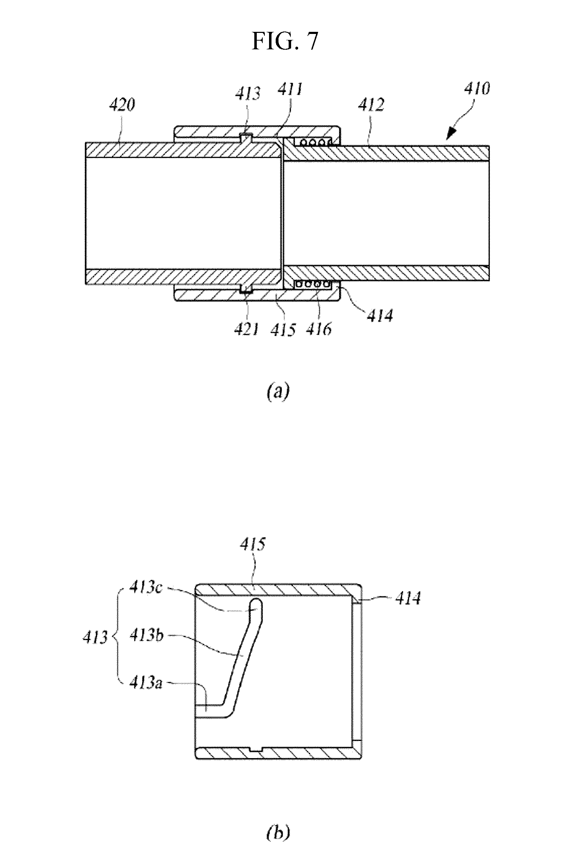

FIG. 6 is an exploded perspective view of a probe according to another embodiment of the present invention, and FIGS. 7A and 7B are cross-sectional views specifically illustrating a detachment unit illustrated in FIG. 6, wherein a groove part of a sleeve is separately illustrated.

Only a connection structure of a probe 400 according to another embodiment of the present invention is different from the probe 200 of FIG. 1, and the other portions of the probe 400 are the same as those of the probe 200 of FIG. 1. In the current embodiment of the present invention, a groove coupling unit 410 is provided to surround a scanhead connector 231 and to be connected to and fixed to one end of a cable assembly 230 and simultaneously, a protrusion coupling unit 420 which is installed in a housing 222 in the vicinity of the board-connection connector 221 and to which the groove coupling unit 410 may be fastened, is provided to correspond to the groove coupling unit 410.

The groove coupling unit 410 includes a tubular connection body 412 which has one end connected to and fixed to the cable assembly 230 and the other end on which an edge part 411 is formed, a sleeve 415 including a groove part 413 which surrounds ends of the connection body 412 and in which a lengthwise groove 413a, a diagonal groove 413b and a circumferential groove 413c are sequentially and successively formed in an inner circumferential surface of the sleeve 415, and a rib 414 formed on an inner circumferential surface of an end of the sleeve 415 adjacent to the cable assembly 230, and an elastic member 416 which is interposed between the sleeve 415 and an outer circumferential surface of the connection body 412 and both ends of which are supported by the rib 414 and the edge part 411.

The edge part 411 limits movement of the sleeve 415 when the sleeve 415 moves in a lengthwise direction of the connection body 412. The groove part 413 of the sleeve 415 is configured in such a way that the lengthwise groove 413a, the diagonal groove 413b, and the circumferential groove 413c are sequentially and successively formed in the inner circumferential surface of the sleeve 415 and thus a protrusion 421 of the protrusion coupling unit 420 that will be described as below moves within the groove part 413 and is fastened to the groove part 413. The rib 414 is formed in a ring shape on the inner circumferential surface of the end of the sleeve 415 and supports one side of the elastic member 416.

The protrusion coupling unit 420 is a tubular member in which the protrusion coupling unit 420 is inserted into an inner side of the sleeve 415 so that an end of the protrusion coupling unit 420 is adjacent to an end of the connection body 412 of the groove coupling unit 410 and at least one protrusion 421 is formed on the outer circumferential surface of the protrusion coupling unit 420 adjacent to the end of the connection body 412 in the radial direction. A board-connection connector 221 is accommodated within the protrusion coupling unit 420.

Thus, when the protrusion coupling unit 420 is inserted into the sleeve 415 of the groove coupling unit 410 and the protrusion 421 moves along the lengthwise groove 413a of the groove part 413, the protrusion coupling unit 420 and the groove coupling unit 410 are adjacent to each other and simultaneously, the board-connection connector 221 and the scanhead connector 231 contact each other. Thereafter, if the user rotates the sleeve 415 manually, the protrusion 421 moves along the diagonal groove 413b of the groove part 413 and is inserted into the circumferential groove 413c so that the groove coupling unit 410 and the protrusion coupling unit 420 can be firmly fastened to and fixed to each other.

When coupling of the groove coupling unit 410 and the protrusion coupling unit 420 is released, if the user rotates the sleeve 415 manually in an opposite direction to a direction in which the groove coupling unit 410 and the protrusion coupling unit 420 are coupled to each other, the protrusion 421 moves along the groove part 413 and escapes from the lengthwise groove 413a. Subsequently, if the user pulls the sleeve 415 in a direction in which the board-connection connector 221 and the scanhead connector 231 are separated from each other, the protrusion coupling unit 420 can be completely separated from the groove coupling unit 410.

The protrusion 421 and the groove part 413 are fastened to each other, and the board-connection connector 221 and the scanhead connector 231 are coupled to each other so that mechanical connection and electrical connection between the scanhead 220 and the cable assembly 230 can be conveniently and firmly performed.

A detachment unit as illustrated in FIG. 3, 4, or 6 may also be provided at a storage cover 240.

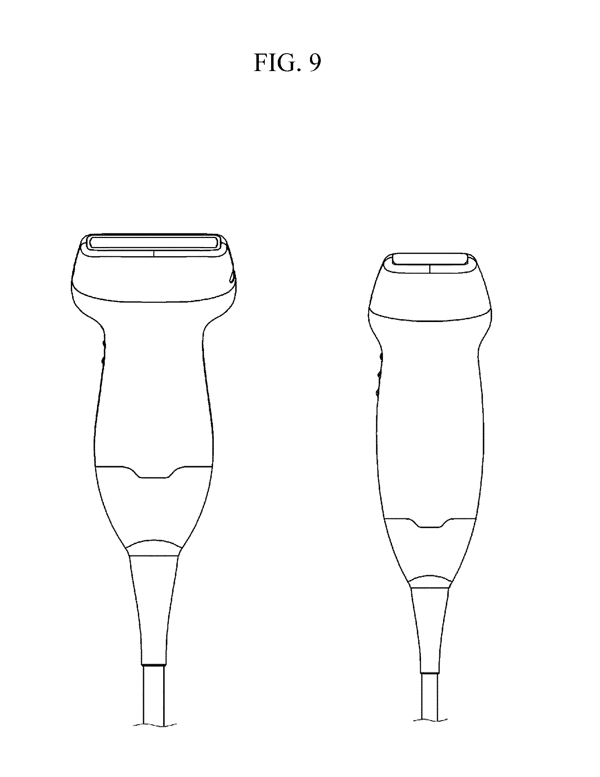

FIG. 9 illustrates part of various types of probes configured according to the present invention. If other types of probes except the probes illustrated in FIG. 9 have the above-described configuration of a scanhead 220 and a board-connection connector 221, the board-connection connector 221 of the scanhead 220 can be coupled to a scanhead connector 231 of a cable assembly 230.

A probe, i.e., a scanhead may have various characteristics, in particular, different frequency bands and may be configured to have different shapes. For example, each scanhead is configured to have one shape selected from the group consisting of a linear shape, a convex shape, and a phased array shape.

The user can select a scanhead suitable for desired performance and a diagnostic region among a plurality of scanheads having different characteristics. That is, according to the present invention, a probe suitable for user's desired performance and a diagnostic region can be selected, and a scanhead corresonding to the selected probe can be connected to the cable assembly. If a different type of diagnostic work is performed while one probe is used, a scanhead of the probe being used is separated from the cable assembly, and instead, a scanhead of another probe suitable for a desired diagnostic work can be connected to the cable assembly.

In the separable probe according to the present invention, the scanhead 220 and the cable assembly 230 are detachably connected to each other via the board-connetion connector 221 and the scanhead connector 231. Thus, if necessary, the scanhead 220 can be separated from the cable assembly 230 such that the scanhead can be replaced with a new one and user's working convenience can be greatly improved.

Furthermore, a degree of freedom at which the scanhead 220 can be designed to be suitable for each type of probe is improved so that an optimized design of the probe can be realized.

In addition, in the probe according to the present invention, only one cable assembly 230 is mounted on a main body unit of an ultrasonic diagnostic system so that the entire structure of the main body unit is simplified, a space in which the probe is to be installed and the size and weight of the entire system can be greatly reduced. In particular, the configuration of the system connector 232 that connects the cable assembly 230 to the main body unit of the ultrasonic diagnostic system is simplified so that the ultrasonic diagnostic system can be easily miniaturized and carried in a portable form.

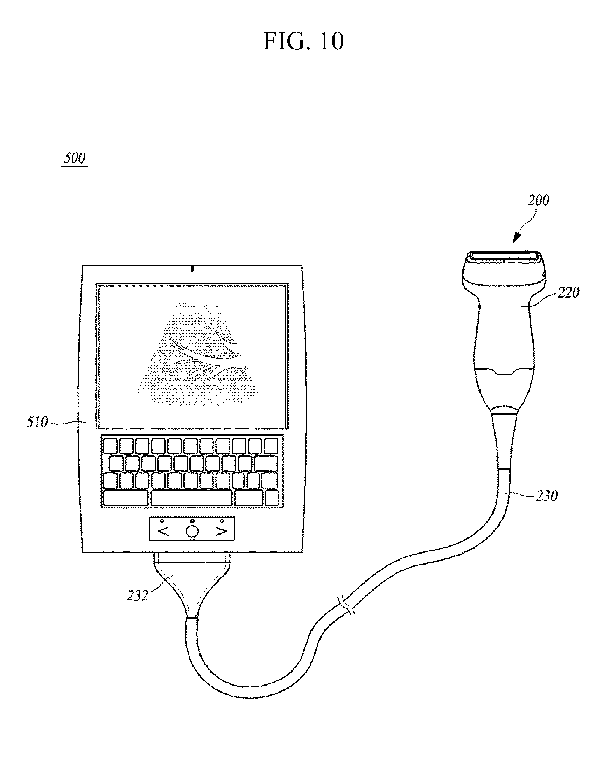

FIG. 10 illustrates an example of a state in which a probe according to the present invention is connected to an ultrasonic diagnostic system. As illustrated in FIG. 10, the probe according to the present invention, for example, the probe 200 illustrated in FIG. 3 enables the size and weight of a main body unit 510 of an ultrasonic diagnostic system 500 to be greatly reduced. This is because only one cable assembly 230 is mounted on the main body unit 510, a tuning board is omitted from the system connector 232 used to connect the cable assembly 230 to the main body unit 510 and thus the configuration of the probe is simplified and the weight of the entire system is reduced and accordingly, the configuration of a socket (not shown) is simplified compared to the related art and the volume of the entire system is greatly reduced compared to the related art.

In the probe according to the present invention, an initial cost for purchasing a cable assembly is not overlappingly required, manufacturing cost of a product can be reduced, and even when maintenance is required, reusable elements can be continuously used without discarding all of the scanhead 220, the cable assembly 230, and the like, thus easily performing maintenance and reducing cost for maintenance.

While the invention has been shown and described with reference to certain exemplary embodiments thereof, it will be understood by those skilled in the art that various changes in form and details may be made therein without departing from the spirit and scope of the invention as defined by the appended claims.

### CROSS-REFERENCE TO RELATED APPLICATION

If the present application claims for a priority on Korean Patent Application No. 10-2010-0116565 filed on November 23, 2010, in Korea under Article 119(a) (35 U.S.C § 119(a)) of the U.S. Patent Act, all contents thereof are integrated with the present application as a reference document. Furthermore, if the present application claims for a priority on other countries than U.S.A on the same grounds as above, all contents thereof are integrated with the present application as a reference document.

## Claims

1. A probe comprising:
a scanhead which includes a housing that functions as a handle, a transducer that is installed at one side of the housing, a tuning board that is accommodated within the housing and has one side connected to a printed circuit board (PCB) of the transducer, and a board-connection connector that is connected to the other side of the tuning board; and
a cable assembly which has one end on which a scanhead connector detachably coupled to the board-connection connector is provided, and has the other end on which a system connector is provided.

2. The probe of claim 1, wherein the board-connection connector and the tuning board are electrically connected to each other using one selected from the group consisting of a press-fit pin structure, a ball grid array (BGA) package structure, and a dip soldering coupling structure.

3. The probe of claim 1, wherein a signal processing circuit including an inductor and an identification (ID) recognition circuit are integrally disposed on the tuning board.

4. The probe of claim 1, wherein a connection cover is formed to extend from the cable assembly so as to surround a periphery of the scanhead connector, and an open part of the connection cover has a shape corresponding to an open part of the housing.

5. The probe of claim 4, wherein a pair of latches are provided at the connection cover, and a pair of hanging grooves or hanging holes which are formed in the housing in a vicinity of the board-connection connector and in which the pair of latches can be hung, are provided to correspond to the pair of latches.

6. The probe of claim 5, wherein the latches further comprise pressurization parts that allow the latches to elastically move by user's pressurization.

7. The probe of claim 4, wherein corresponding stepped parts are formed at front ends of the open parts.

8. The probe of claim 4, wherein the connection cover is manufactured using double injection or insert injection using a waterproof material.

9. The probe of claim 1, further comprising:
a ball coupling unit that is provided to surround the scanhead connector and to be connected to and fixed to one end of the cable assembly; and
a snap coupling unit which is installed in the housing in a vicinity of the board-connection connector and to which the ball coupling unit is able to be fastened.

10. The probe of claim 9, wherein the ball coupling unit comprises a tubular coupling body which has one end connected to and fixed to the cable assembly and has the other end on which an edge part is formed and in which support protrusions are formed on an outer circumferential surface of the coupling body to be spaced apart from the edge part and a plurality of through holes are formed in a space between the edge part and the support protrusions, ball members that are mounted in the plurality of through holes of the coupling body, a sleeve including a first stepped part and a second stepped part on an inner circumferential surface while surrounding ends of the coupling body, and an elastic member that is interposed between the second stepped part of the sleeve and an outer circumferential surface of the coupling body and is elastically supported by the support protrusions, and
the snap coupling unit is a tubular member in which the snap coupling unit is inserted into an inner side of the coupling body of the ball coupling unit and a ring-shaped coupling groove is formed adjacent to an end of the snap coupling unit in a circumferential direction.

11. The probe of claim 10, wherein the edge part or the support protrusions are replaceable with a C-shaped ring.

12. The probe of claim 10, wherein diameters of the through holes of the coupling body toward an inner circumferential surface of the coupling body are formed to be small, and diameters of the through holes of the coupling body toward the outer circumferential surface of the coupling body are formed to be relatively large and thus the ball members do not pass through the through holes but are mounted in the through holes while parts of the ball members protrude toward the inner circumferential surface of the coupling body, the first stepped part provides a clearance in which the ball members are movable from the through holes when the sleeve moves, and an end of the sleeve toward the first stepped part covers the through holes of the coupling body when the sleeve moves to the maximum.

13. The probe of claim 1, further comprising:
a groove coupling unit that is provided to surround the scanhead connector and to be connected to and fixed to one end of the cable assembly; and
a protrusion coupling unit which is installed in the housing in a vicinity of the board-connection connector and to which the groove coupling unit is able to be fastened.

14. The probe of claim 13, wherein the groove coupling unit comprises a tubular connection body which has one end connected to and fixed to the cable assembly and the other end on which an edge part is formed, a sleeve including a groove part which is formed in an inner circumferential surface of the sleeve and a rib formed on an inner circumferential surface of an end of the sleeve adjacent to the cable assembly while surrounding ends of the connection body, and an elastic member which is interposed between the sleeve and an outer circumferential surface of the connection body and both ends of which are supported by the rib and the edge part, and
the protrusion coupling unit is a tubular member in which the protrusion coupling unit is inserted into an inner side of the sleeve in a state in which an end of the protrusion coupling unit comes into contact with an end of the connection body of the groove coupling unit and at least one protrusion is formed on the outer circumferential surface of the protrusion coupling unit adjacent to the end of the connection body in a radial direction.

15. The probe of claim 14, wherein the groove part of the sleeve is configured in such a way that a lengthwise groove, a diagonal groove, and a circumferential groove are sequentially and successively formed in the inner circumferential surface of the sleeve and thus a protrusion of the protrusion coupling unit moves within the groove part and is fastened to or released from the groove part.

16. The probe of claim 1, wherein a rubber ring or gasket is disposed in a portion in which the board-connection connector and the scanhead connector are coupled to each other.

17. The probe of claim 1, wherein a system connector is provided on the other end of the cable assembly, and the system connector is connected to a socket mounted in a main body unit of an ultrasonic diagnostic system and having a structure corresponding to the system connector.

18. The probe of claim 1, wherein the board-connection connector, the scanhead connector, and the system connector comprise units for preventing misinsertion.

19. The probe of claim 1, further comprising a storage cover that is coupled to the open part of the housing when the cable assembly is separated from the scanhead.
